(19) Europäisches Patentamt
European Patent Office
Office européen des brevets

(11) **EP 2 553 455 B1**

(12) **EUROPÄISCHE PATENTSCHRIFT**

(45) Veröffentlichungstag und Bekanntmachung des Hinweises auf die Patenterteilung:
**24.01.2018 Patentblatt 2018/04**

(21) Anmeldenummer: **11715169.6**

(22) Anmeldetag: **01.04.2011**

(51) Int Cl.:
*G01N 33/543* (2006.01)    *A61B 5/05* (2006.01)
*G01R 33/12* (2006.01)    *G01N 33/569* (2006.01)
*G01R 33/00* (2006.01)    *A61B 5/055* (2006.01)
*A61B 5/00* (2006.01)    *C12Q 1/04* (2006.01)
*G01N 33/68* (2006.01)

(86) Internationale Anmeldenummer:
**PCT/EP2011/001650**

(87) Internationale Veröffentlichungsnummer:
**WO 2011/120712 (06.10.2011 Gazette 2011/40)**

(54) **VERFAHREN ZUR BESTIMMUNG DER ANWESENHEIT EINES ANALYTEN MITTELS MAGNETISCHER KLEINSTPARTIKEL SOWIE VORRICHTUNG HIERFÜR**

METHOD FOR DETERMINING THE PRESENCE OF AN ANALYTE BY MEANS OF SMALL MAGNETIC PARTICLES, AND CORRESPONDING DEVICE

PROCÉDÉ DE DÉTERMINATION DE LA PRÉSENCE D'UN ANALYTE PAR DE PETITES PARTICULES MAGNÉTIQUES ET DISPOSITIF ADAPTÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2010 DE 102010013900**

(43) Veröffentlichungstag der Anmeldung:
**06.02.2013 Patentblatt 2013/06**

(73) Patentinhaber:
• **Hochschule Für Angewandte Wissenschaften Fachhochschule Würzburg-Schweinfurt 97070 Würzburg (DE)**
• **Julius-Maximilians-Universität Würzburg 97070 Würzburg (DE)**

(72) Erfinder:
• **RÜCKERT, Martin 97234 Reichenberg (DE)**
• **BEHR, Volker, C. 97218 Gerbrunn (DE)**
• **KAMPF, Thomas 97072 Würzburg (DE)**

(74) Vertreter: **FDST Patentanwälte Nordostpark 16 90411 Nürnberg (DE)**

(56) Entgegenhaltungen:
WO-A1-2009/037636    WO-A1-2010/041178
WO-A2-2009/008956    US-A1- 2004 033 627
US-A1- 2010 066 363

• **H. CARR ET AL: "Effects of Diffusion on Free Precession in Nuclear Magnetic Resonance Experiments", PHYSICAL REVIEW, Bd. 94, Nr. 3, 1. Mai 1954 (1954-05-01), Seiten 630-638, XP55004628, ISSN: 0031-899X, DOI: 10.1103/PhysRev.94.630**

**Beschreibung**

Die Erfindung betrifft ein neuartiges Verfahren zur Bestimmung der Anwesenheit eines Analyten mittels einer Verteilung magnetischer Kleinstpartikel. Weiterhin betrifft die Erfindung eine zur Durchführung dieses Verfahrens geeignete Vorrichtung.

Ein Verfahren der eingangs genannten Art ist aus der US 2008/0220411 A1 bekannt. Dort wird zur Bestimmung der Anwesenheit eines Analyten, beispielsweise eines Bakteriums, eines Virus, einer biologischen Zelle, eines Proteins oder einer Nukleinsäure, die asynchrone Rotation der Magnetisierung eines entsprechend präparierten magnetischen Kleinstpartikels in einem rotierenden äußeren Magnetfeld verwendet. Dem offenbarten Verfahren liegt die Tatsache zugrunde, dass die Rotation der magnetischen Kleinstpartikel oder deren Magnetisierung in einem äußeren rotierenden Magnetfeld abhängig von Feldstärke und Rotationsfrequenz in einen Bereich synchroner und in einen Bereich asynchroner Drehung zerfällt.

Im Falle eines mechanisch rotierenden Kleinstpartikels liegt dieses Verhalten an Reibungstermen gegenüber der Umgebung. Rotiert die Magnetisierung im Kristallgitter des Kleinstpartikels, so sind andere, innere Reibungsterme verantwortlich. Ist das vom äußeren Magnetfeld übertragene Drehmoment gegenüber den Reibungstermen zu gering, so bleibt die Magnetisierung bzw. das Kleinstpartikel gegenüber der Rotation des Magnetfeldes zurück, da es gegenüber der Umgebung verharren möchte. Es entsteht eine nicht-lineare Fluktuation der Drehung der Magnetisierung gegenüber der Rotation des Magnetfeldes. Im Mittel ergibt sich dabei eine messbare Rotationsdrift.

Entsprechend der US 2008/0220411 A1 rotiert die Magnetisierung eines Kleinstpartikels abhängig von der Feldstärke des äußeren Magnetfeldes im Mittel bis zu einer kritischen Frequenz $\Omega_C$ synchron zur Rotation des Magnetfeldes.

Überschreitet die Rotationsfrequenz des Magnetfeldes die kritische Rotationsfrequenz $\Omega_C$, so dreht die Magnetisierung des Kleinstpartikels mit einer verringerten mittleren Rotationsfrequenz asynchron. Diese mittlere, asynchrone Drehung der Magnetisierung kann als ein Frequenzterm der Magnetisierung beobachtet werden. Bei kleinerer Feldstärke ergibt sich eine niedrigere kritische mittlere Rotationsfrequenz $\Omega_C$ als bei größerer Feldstärke. Der Zusammenhang zwischen kritischer mittlerer Rotationsfrequenz $\Omega_C$ und der Feldstärke B des äußeren Magnetfeldes ist dazu gegeben durch:

$$\Omega_c = \frac{mB}{\gamma} .$$

Dabei bezeichnet m das magnetische Moment des Kleinstpartikels. Der Term $\gamma$ beschreibt einen Reibungsterm, der im Falle einer mechanischen Rotation des Kleinstpartikels die Viskosität der Umgebung und einen Formfaktor des Kleinstpartikels beinhaltet. Im Falle einer Rotation der Magnetisierung im Kristallgitter des Kleinstpartikels ist der Reibungsterm $\gamma$ empirisch zu ermitteln.

Die US 2008/0220411 A1 berücksichtigt nun die Tatsache, dass sich die nicht-lineare Rotationsdrift bei asynchroner Rotation der Kleinstpartikel abhängig von deren Bindungs- oder Kopplungzustand ändert. Wird ein magnetisches Kleinstpartikel beispielsweise an seiner Oberfläche so präpariert, dass es an einen interessierenden Analyten anbindet, so kann auf die Anwesenheit des Analyten geschlossen werden, wenn eine Änderung der nicht-linearen Rotationsdrift zu beobachten ist. Das gekoppelte Kleinstpartikel weist gegenüber dem freien Kleinstpartikel beispielsweise aufgrund seiner Größenzunahme eine verringerte asynchrone Rotationsfrequenz auf. Die US 2008/0220411 A1 lehrt weiter, die Rotationsfrequenz der Kleinstpartikel durch deren direkte Beobachtung optisch zu messen. Dazu wird beispielsweise lediglich eine Hemisphäre der Kleinstpartikel entsprechend beschichtet, so dass die Rotationsfrequenz aus dem Wechsel der beiden Hemisphären unmittelbar beobachtet werden kann.

Nachteiligerweise ist das Verfahren gemäß Stand der Technik eingeschränkt auf die Beobachtung einer makroskopischen Drehung des gebundenen magnetischen Kleinstpartikels. Die optische Messung ist aufwändig und fehlerbehaftet. Dokument WO 2009/037636 beschreibt eine Vorrichtung zur Messung unter anderem der Bindungseigenschaften magnetischer Partikel mittels rotierender Magnetfelder. Aufgabe der Erfindung ist es, ein Verfahren zur Feststellung der Anwesenheit eines Analyten mittels magnetischer Kleinstpartikel anzugeben, mit dem gegenüber dem Stand der Technik zusätzliche Anwendungen erschlossen werden können. Eine weitere Aufgabe der Erfindung ist es, eine zur Durchführung dieses Verfahrens geeignete Vorrichtung anzugeben. Die Aufgabe wird durch ein Verfahren gemäß des Anspruchs 1 gelöst. Die Erfindung geht dabei in einem ersten Schritt von der Überlegung aus, die asynchrone Rotation der Kleinstpartikel nicht mehr optisch, sondern über die superpositionierte Quermagnetisierung des Partikelensembles zu erfassen. In einem äußeren rotierenden Magnetfeld sind die Magnetisierungen der Kleinstpartikel makroskopisch ausgerichtet und rotieren um die Rotationsachse des Magnetfeldes. In einer Projektion auf eine Ebene senkrecht zur Rotationsachse rotiert die superpositionierte Quermagnetisierung. Rotieren die Magnetisierungen der Kleinstpartikel in Phase, so resultiert eine maximale superpositionierte Quermagnetisierung, die mit der Rotationsfrequenz der Kleinstpartikel rotiert. Geraten die Magnetisierungen der Kleinstpartikel außer Phase, so verschwindet die beobachtbare Quermagnetisierung mit einer charakteristischen Verfallsdauer. Sind die Magnetisierungen der Kleinstpartikel stochastisch ausgerichtet, so ist die superpositionierte Quermagnetisierung nicht mehr beobachtbar. Im Mittel heben sich alle Magnetisierungen der Kleinstpartikel dann auf.

Ist das rotierende äußere Magnetfeld derart gewählt, dass die Magnetisierungen der Kleinstpartikel asynchron rotieren, so ist die Messfrequenz, nämlich die Rotationsfrequenz der superpositionierten Quermagnetisierung, von der Erregerfrequenz, nämlich der Rotationsfrequenz des äußeren Feldes, getrennt. Das Erregersignal spricht nicht auf das Messsignal über. Die Empfindlichkeit der Messung wird hierdurch deutlich verbessert. Aufwändige Methoden zur Abtrennung des Erregersignals vom Messsignal können entfallen. Zu einer wirksamen Abtrennung genügt ein einfacher Bandpass-Filter oder Tiefpass-Filter.

In einem zweiten Schritt geht die Erfindung von der Erkenntnis aus, dass zur Beobachtung der superpositionierten Quermagnetisierung ein definierter Ausgangszustand des beobachteten Systems geschaffen werden muss. Dieser Ausgangszustand muss sich zumindest von einem Zustand des Systems unterscheiden, in dem die magnetischen Momente der Kleinstpartikel stochastisch verteilt sind. Demnach muss eine definierte Störung in das System eingebracht werden, so dass sich die Magnetisierungen der Kleinstpartikel zumindest zu einer beobachtbaren superpositionierten Quermagnetisierung addieren. Dazu wird in einem Präparationsschritt zu Beginn jeder Messung ein äußeres magnetisches Fokussierungsfeld erzeugt, wodurch wenigstens ein Teil der Magnetisierungen der Kleinstpartikel zueinander ausgerichtet wird. Wird nach Abschalten des Fokussierungsfeldes ein rotierendes äußeres Magnetfeld entsprechender Stärke und Frequenz eingeschaltet, so rotieren die ausgerichteten Magnetisierungen oder magnetischen Momente der Kleinstpartikel zunächst ausgehend von dem definierten Startzustand in Phase, so dass eine superpositionierte Quermagnetisierung zu beobachten ist. Erfindungsgemäß werden die Frequenzanteile der superpositionierten Quermagnetisierung des Partikelensembles erfasst. Diese Frequenzanteile entsprechen insbesondere den Rotationsfrequenzen der Magnetisierungen der Kleinstpartikel. Rotieren die Magnetisierungen der Kleinstpartikel alle in Phase, so entspricht die Rotationsfrequenz der superpositionierten Quermagnetisierung der mittleren Rotationsfrequenz der Magnetisierungen der Kleinstpartikel. Durch Feldinhomogenitäten, durch Streuung der Partikeleigenschaften und durch Unterschiede in der jeweiligen chemischen Umgebung zeigen die Magnetisierungen der Kleinstpartikel eine unterschiedliche asynchrone Rotationsdrift, was zu einer zunehmenden Dephasierung führt. In der superpositionierten Quermagnetisierung sind verschiedene Frequenzanteile erfassbar.

Über eine Analyse der Frequenzanteile der Quermagnetisierung wird es möglich, auf die Anwesenheit eines Analyten zu schließen. Koppelt beispielsweise ein präpariertes Kleinstpartikel als Sonde an den Analyten an, oder bindet sich der Analyt über Rezeptoren an die Oberfläche des Kleinstpartikels, so ändert sich über die Größe und die Form des behafteten Kleinstpartikels auch seine asynchrone Rotationsdrift. Mittels einer Analyse der Frequenzanteile der superpositionierten Quermagnetisierung wird es möglich, auf die Anwesenheit des Analyten zu schließen.

Desweiteren lässt sich über die Analyse der Frequenzanteile der Quermagnetisierung auf die physikalischen und/oder chemischen Umgebungsparameter des Analyten schließen. Insbesondere führen z.B. Änderungen des pH-Wertes, der Temperatur oder der Viskosität der Umgebung des Analyten zu Änderungen in den Frequenzanteilen des detektierten Signals.

Andererseits kann als Zeitverlauf der Quermagnetisierung auch die Änderung ihrer betragsmäßigen Größe beobachtet werden. Die anfangs bei gleichphasig rotierenden Magnetisierungen der Kleinstpartikel maximale Quermagnetisierung zerfällt nämlich nicht nur aufgrund der beschriebenen Feldinhomogenitäten und der vorhandenen Streuung der Partikeleigenschaften, sondern insbesondere auch aufgrund von Diffusions- und Reibungstermen. Bei der sogenannten Brown-Relaxation rotieren die magnetischen Kleinstpartikel mechanisch. Die Relaxation ist hierbei von der Größe des Kleinstpartikels und im Wesentlichen von der Viskosität des umgebenden Mediums abhängig. Bei der Neel-Relaxation rotiert die Magnetisierung der Kleinstpartikel im Kristallgitter. Die Relaxation ist hierbei abhängig von inneren, empirisch ermittelten Reibungstermen. Koppelt erfindungsgemäß ein Kleinstpartikel mit einem Analyten, so ändert sich infolgedessen die beobachtbare Relaxationszeit. Bei Kleinstpartikeln mit Teilchendurchmessern im Bereich einiger 100 nm gilt hierbei als Faustformel, dass freie Kleinstpartikel gegenüber gebundenen oder angekoppelten Kleinstpartikeln eine schnellere Relaxation der superpositionierten Magnetisierung zeigen. Durch eine Beobachtung des Abklingverhaltens der superpositionierten Quermagnetisierung kann insofern wiederum unmittelbar auf die Anwesenheit eines Analyten geschlossen werden.

Findet in Anwesenheit des Analyten eine Verclusterung der Kleinstpartikel statt, so kann auch dies über den Abfall der superpositionierten Quermagnetisierung beobachtet werden. In diesem Fall zerfällt nämlich die nach der Präparation der Probe mittels des Fokussierungsfelds erzeugte makroskopische Magnetisierung bei Anlegen des äußeren rotierenden Magnetfelds aufgrund der gegebenen mechanischen und magnetischen Kopplungen der Einzelpartikel im Cluster beachtlich schnell. Dieser Zerfall kann unmittelbar mit Einschalten des rotierenden äußeren Magnetfelds durch einen pulsartigen Verlauf der superpositionierten Quermagnetisierung beobachtet werden.

Durch Kalibrierungen der aufgenommenen Messkurven der Quermagnetisierung wird es sogar möglich, weitere Informationen über den Analyten zu gewinnen. Derartige Informationen sind beispielsweise die Konzentration oder die Art des Analyten.

Als magnetische Kleinstpartikel werden ferromagnetische oder superparamagnetische Partikel eingesetzt, die im Verhältnis zu ihrem Volumen ein möglichst großes magnetisches Moment aufweisen. Solche Partikel können z.B. aus Eisenoxid bestehen, aber auch bspw. Kobalt, Nickel oder Gadolinium enthalten. Für einen Einsatz in der Biologie und in der Medizin kommen insbesondere magnetische Nanopartikel mit Teilchendurchmessern zwischen 20 und 200 nm in Frage. Je größer das Verhältnis zwischen magnetischem Moment und Volumen ist, desto höher ist im Falle der asynchronen Rotation die Rotationsdrift gegenüber dem äußeren magnetischen Feld.

Die Längs- oder Rotationsachse, um die das äußere Magnetfeld rotiert, muss für die Erfindung nicht zwangsläufig im Raum festliegen. Vielmehr kann die Rotationsachse des Magnetfeldes räumlich auch variieren, was vom beanspruchten Gegenstand ebenfalls umfasst ist.

Um in der Medizintechnik die Verträglichkeit der Kleinstpartikel zu erhöhen, wird die Partikeloberfläche mit einem Coating versehen. Das Coating kann beispielsweise aus Dextranmolekülen bestehen. Für die Verwendung der Kleinstpartikel als Sonden werden der Partikeloberfläche verschiedene funktionale Gruppen angelagert. Diese funktionalen Gruppen binden spezifisch an den Analyten, so dass die Analyse speziell auf einen bestimmten Analyten eingestellt werden kann.

Als Zielsubstanz bzw. als Analyt wirken beispielsweise Rezeptoren von Makrophagen, Biomolekülen oder Zellen, weiter Viren, Bakterien, Moleküle, Makromoleküle, Antigene oder Antikörper. Bindet der Analyt an die funktionalisierte Oberfläche der magnetischen Kleinstpartikel, so wird die mechanische Rotation im äußeren rotierenden Magnetfeld relativ schwach gehemmt. Ist der zu beobachtende Analyt zweifach oder mehrfach bindend, so bilden sich Aggregate aus den Kleinstpartikeln, was zu einer relativ starken Hemmung der mechanischen Rotation führt. Bindet der Analyt auch an nichtmagnetische Kleinstpartikel, so ändern die entstehenden Aggregate ihr hydrodynamisches Volumen. Das magnetische Moment des einzelnen Partikels bleibt jedoch bestehen. Als Folge findet eine weiter verstärke Hemmung der mechanischen Partikelrotation statt.

Sind Ziel der Analyse bindende Gruppen an Zellwänden oder an Objekten, die erheblich größer als das bindende Kleinstpartikel sind, so wird die mechanische Rotation in der asynchronen Domäne effektiv verlangsamt oder behindert. Gegebenenfalls ist dann eine Rotation der Magnetisierung im Kristallgitter zu beobachten.

In einer besonders vorteilhaften Ausgestaltung wird mittels des beschriebenen Verfahrens eine Biopsie an einem physiologischen oder pathologischen Gewebe oder eine Untersuchung an Blut oder anderen entnommenen Körperflüssigkeiten durchgeführt. Dabei werden magnetische Kleinstpartikel mit einer präparierten Partikeloberfläche eingesetzt, die an Rezeptoren von pathologischen Zellen oder Pathogenen binden. In einem Zielvolumen von lediglich einigen $\mu l$ kann insbesondere direkt eine Biopsienadel mit einem Durchmesser von weniger als 1 mm oder eine kleine Flüssigkeitsprobe analysiert werden. Das Verfahren erlaubt eine rasche und einfache Überprüfung dahingehend, ob die entnomme Probe pathologisch ist. Hiermit lassen sich Erkrankungen wie z.B. Tuberkulose über die Bindung der Partikel an das Mycobacterium tuberculosis in einer Probe des Auswurfs des Patienten einfach und schnell nachweisen.

In einer bevorzugten Ausgestaltung wird der zeitliche Verlauf der Quermagnetisierung erst mit einer Verzögerungszeit nach dem Abschalten des Fokussierungsfeldes erfasst. Dabei wird dem Umstand Rechnung getragen, dass bei den hier relevanten Kleinstpartikeln die makroskopische Magnetisierung eines Ensembles ausgerichteter freier Kleinstpartikel rascher relaxiert als die makroskopische Magnetisierung eines Ensembles ausgerichteter gebundener Kleinstpartikel. Wird zwischen der Präparation der Probe und dem auslesenden äußeren rotierenden Magnetfeld eine Verzögerungszeit eingebracht, so können die freien Kleinstpartikel aus dem Messsignal separiert werden. Wird das rotierende äußere Magnetfeld nach einer Verzögerungszeit eingeschaltet, die größer ist als die Relaxationszeit der Magnetisierung der freien Kleinstpartikel, so tragen nur noch die Magnetisierungen der gebundenen Kleinstpartikel zum Betrag der superpositionierten Quermagnetisierung bei. Die Magnetisierungen der freien Kleinstpartikel sind zum Zeitpunkt der Messung stochastisch verteilt, so dass sich deren superpositionierte Quermagnetisierung zu Null addiert. Die Selektivität und die Empfindlichkeit der Messmethode werden hierdurch verbessert.

Zweckmäßigerweise erfolgt die Erfassung des zeitlichen Verlaufs der Quermagnetisierung erst nach einer Totzeit nach dem Abschalten des Fokussierungsfeldes. Auf diese Weise wird messtechnisch verhindert, dass das starke Erregersignal des Fokussierungsfeldes beispielsweise induktiven Eingang in das Messsignal findet. Erfolgt die Messung der superpositionierten Quermagnetisierung insbesondere induktiv, so wird während der Totzeit die Messspule idealer Weise kurzgeschlossen oder getrennt, um ein langes Nachschwingen des Fokussierungspulses im Tiefpass zu unterdrücken.

In einer weiter vorteilhaften Ausgestaltung wird aus dem Verlauf der Quermagnetisierung die zeitliche Ableitung gebildet, und aus der Ableitung auf die Anwesenheit des Analyten geschlossen. Hierdurch werden zeitliche Änderungen in der Quermagnetisierung leichter sichtbar. Insbesondere erfolgt bei einer induktiven Messaufnahme eine zeitliche Ableitung der Quermagnetisierung infolge der Messmethode.

Weiter vorteilhaft wird die Rotationsrichtung des rotierenden Magnetfeldes mit einer Echofrequenz in der Richtung umgekehrt. Ähnlich dem Spin-Echo aus der Kernspintomographie lässt sich durch eine Richtungsumkehr des äußeren rotierenden Magnetfeldes die Dephasierung der asynchron rotierenden Kleinstpartikel umkehren. Nach Umkehr der Rotationsrichtung gerät zumindest ein Teil der Magnetisierungen der Kleinstpartikel erneut in Phase, was sich in einem Anwachsen des Signals der superpositionierten Quermagnetisierung zeigt. Die Anwendung des Echo-Verfahrens führt aufgrund der wiederholten Signalaufnahme zu einem verbesserten Signal-Rausch-Verhältnis. In der Zeitdomäne wird ein verbreitertes Messsignal aufgenommen, so dass die Bandbreite des Signals im Frequenzraum verringert und somit messtechnisch optimiert ist. Über eine Beobachtung der Abklingrate der Echo-Signale kann ferner auf die Struktur der gebildeten gebundenen Partikel geschlossen werden. So drehen beispielsweise anisotrope Partikel aus der Rotationsebene des Magnetfelds heraus, so dass die hierdurch bedingte Dephasierung auch durch Echo-Verfahren nicht mehr rückgängig gemacht werden kann.

Das rotierende Magnetfeld kann beispielsweise mit einer in Umfangsrichtung angeordneten Anzahl von einzeln bestrombaren Wicklungen oder Spulenanordnungen erzeugt werden. Dabei kann das Drehfeld insbesondere ein- oder mehrphasig erzeugt werden. In einer konstruktiv einfachen und kostengünstigen Variante wird das rotierende Magnetfeld mittels zweier senkrecht zueinander stehender Spulen-Paare, insbesondere Helmholtz-Spulenpaare, erzeugt. Durch abwechselnde Bestromung der Spulenpaare wird das rotierende Magnetfeld erzeugt. Über die Taktung der Bestromung wird die Rotationsfrequenz gesteuert. Der durch die Spulen fließende Strom steuert die Stärke des rotierenden Magnetfeldes.

In einer weiter vorteilhaften Variante werden die beiden Spulenpaare mit zeitlich variierenden Phasendifferenzen angesteuert. Dies kann mittels sich unterscheidender Frequenzen und/oder sich unterscheidender Phasen vorgenommen werden. Durch eine derartige Ansteuerung kann erreicht werden, dass das Feld des einen Spulenpaares zeitlich betrachtet gewissermaßen das andere Feld mehrfach überholt, so dass mit einer Schwebungsfrequenz der beiden anliegenden Frequenzen eine Drehrichtungsumkehr des erzeugten rotierenden Magnetfeldes erfolgt. Durch eine solche Ansteuerung kann relativ einfach eine periodisch wiederkehrende Drehrichtungsumkehr des rotierenden Magnetfeldes vorgenommen werden, so dass die Aufnahme der superpositionierten Quermagnetisierung gemäß dem Echo-Verfahren erfolgt.

Das Fokussierungsfeld kann durch Einschalten eines entsprechenden Magnetfeldgenerators erzeugt werden. Da ein scharfer Rechteckpuls im Frequenzraum unerwünscht viele Seitenbanden (die Harmonischen höherer Ordnung) generiert, erfolgt das Ein- und Ausschalten des Fokussierungsfelds bevorzugt mittels eines Gauß- oder mittels eines Sinc-Pulses oder mittels einer Linearkombination von Gauß- und/oder Sinc-Pulsen. Da sich die Frequenzanteile eines Gauß-Pulses nur um die Zentralfrequenz verteilen, ist es möglich, das Erregersignal vom Messsignal durch einen Tiefpass zu trennen. Ein Sinc-Puls, das heißt ein Puls der Funktion $\sin(t)/t$, stellt die Fouriertransformierte eines Rechteckpulses dar. Insofern besitzt ein Sinc-Puls im Frequenzraum eine erheblich schärfer abgegrenzte Bandbreite als der Gauss-Puls. Kontinuierliche bandbreitenlimitierte Signale lassen sich insbesondere als Linearkombination von Sinc-Pulsen darstellen.

Ein gerichtetes Fokussierungsfeld führt zu einer Ausrichtung der Magnetisierungen der Kleinstpartikel und somit zu einer ausgerichteten makroskopischen Magnetisierung. Zur Generierung eines derartigen Fokussierungsfelds kann bevorzugt auch der Magnetfeldgenerator verwendet werden, der zur Erzeugung des äußeren rotierenden Magnetfelds eingesetzt ist. Beispielsweise wird hierfür nur ein Spulenpaar oder eine Spule der eingesetzten Spulenanordnungen entsprechend angesteuert.

In einer anderen Variante wird das Fokussierungsfeld durch das rotierende Magnetfeld selbst erzeugt. Dabei genügt insbesondere das Einschalten des rotierenden Magnetfeldes, um eine messbare Störung der stochastisch ausgerichteten Magnetisierungen der Kleinstpartikel zu erzielen. Vorteilhafterweise wird das Fokussierungsfeld durch eine Erhöhung der Feldstärke des rotierenden Magnetfelds erzeugt. Durch die Erhöhung der Feldstärke werden die Magnetisierungen der Kleinstpartikel zu einer synchronen Rotation mit dem äußeren Magnetfeld gezwungen. Alle Magnetisierungen der Kleinstpartikel rotieren dann in Phase mit der Frequenz des Rotationsfeldes. Durch nachfolgendes Absenken der Feldstärke bis in den Bereich der asynchronen nicht-linearen Rotation erfolgt dann das Auslesen der präparierten Probe. Zwar ist die Bereitstellung eines Fokussierungsfeldes auch durch Absenken der Rotationsfrequenz des äußeren rotierenden Magnetfeldes möglich, jedoch wird hierdurch der Abstand zwischen der Erregerfrequenz und der Messfrequenz in messtechnisch unerwünschter Weise verringert.

In einer weiter vorteilhaften Ausgestaltung wird das Refokussierungsfeld derart erzeugt, dass selektiv nur die Magnetisierungen von Kleinstpartikeln gleicher Rotationseigenschaften in eine synchrone Rotation versetzt werden. Dabei wird die Tatsache ausgenutzt, dass die beschriebene kritische Frequenz $\Omega_c$, die den Übergang zwischen synchroner und asynchroner Rotation beschreibt, auch vom Volumen bzw. der Form der Kleinstpartikel abhängig ist. Diese Abhängigkeit führt zum einen dazu, dass das Messsignal der erfassten superpositionierten Quermagnetisierung sich bei zunehmender Bandbreite der Kleinstpartikelvariation verschlechtert. Wird auf der anderen Seite der Refokussierungspuls so erzeugt, dass nur Kleinstpartikel gleicher Rotationseigenschaften in eine synchrone Rotation versetzt werden, so tragen nur diese zur superpositionierten Quermagnetisierung bei. Die Magnetisierungen der anderen Kleinstpartikel sind hingegen stochastisch verteilt. Insgesamt wird auf diese Weise eine Verbesserung des erfassten Messsignals erhalten, ohne dass die Bandbreite der Größen der eingesetzten Kleinstpartikel verringert werden muss, was gegebenenfalls mit hohen Kosten verbunden ist.

Bei Partikeln mit Neelrelaxation ist effektiv die Größe das entscheidende Selektionskriterium. Entscheidend ist generell das Verhältnis aus magnetischem Moment des Partikels und seinem Reibungsterm. Bei massiven Partikeln mit im Gitter fixierter Magnetisierung ist der Reibungsterm unabhängig von der Größe, sondern hängt nur vom Formfaktor ab. Durch die geeignete Wahl der Feldstärke und/oder der Rotationsfrequenz des Refokussierungsfeldes werden demnach Partikel gleicher Rotationseigenschaften, d.h. Partikel mit gleicher Rotationsdrift bei gleicher Feldstärke und gleicher Rotationsfrequenz, selektiert.

Eine solche selektive Refokussierung kann beispielsweise durch ein rotierendes Magnetfeld erzielt werden, dessen Drehachse auf einer Trajektorie umläuft. In diesem Fall gibt es nur für ganz bestimmte Partikel mit einem ganzzahligen Teilerverhältnis ihrer Rotationsfrequenz eine Refokussierung. Denn nur solche Partikel gelangen nach Abschluss der Anwendung des 3D-Rotationsfeldes wieder in ihre Ausgangsposition zurück, die eine 1/n-fache Rotationsfrequenz gegenüber der Rotation des Refokussierungsfeldes aufweisen (n: ganzzahlig). Auf diese Weise können hochselektive Refokussierungspulse erreicht werden. Die Signalselektion erfolgt durch einen geeigneten Tief- oder Bandpassfilter.

Durch eine Vergleichsmessung mit einer definierten Probe wird es möglich, aus dem erfassten zeitlichen Verlauf der Quermagnetisierung auf die Konzentration des Analyten zu schließen. Grundsätzlich gilt hierbei, dass der Betrag der Quermagnetisierung mit der Anzahl der gekoppelten Kleinstpartikel im Verhältnis zu ihrer Gesamtanzahl steigt.

In einer bevorzugten Ausgestaltung wird das Fokussierungsfeld zwischen 10 ns und 10 ms mit Feldstärken zwischen 0,1 mT und 1 T erzeugt, um eine Ausrichtung oder eine Rotation der makroskopischen Magnetisierung bzw. der superpositionierten Quermagnetisierung zu erreichen. Dabei gilt die längere Pulsdauer für das schwächere Feld und die kürzere Pulsdauer für das stärkere Feld. Beide Varianten stellen unterschiedliche Anforderungen an die Signalkette. Bei Probengrößen von wenigen $\mu$l und Einschaltzeiten von wenigen $\mu$s sind auch Felder von 1 T relativ leicht handhabbar. Für eine asynchrone nicht-lineare Rotation der bevorzugt eingesetzten Nano-Partikel weist das rotierende Magnetfeld vorteilhafterweise Feldstärken zwischen 0,1 mT und 100 mT und Frequenzen von 100 Hz bis 100 MHz auf. Die hohen Frequenzen kommen insbesondere bei kleinen Probengrößen in Frage, da keine physiologischen Grenzwerte eingehalten werden müssen.

In einer weiter bevorzugten Ausgestaltung wird über ein für eine vorgegebene Zeitspanne eingeschaltetes Gradientenfeld dem Partikelensemble eine ortsabhängige Phasenlage eingeprägt. Solange das magnetische Gradientenfeld eingeschaltet ist, rotieren die einzelnen magnetischen Kleinstpartikel ortsabhängig mit jeweils unterschiedlichen asynchronen Drehfrequenzen, woraus sich nach dem Abschalten eine ortsabhängige Phasenlage ergibt. Das Gradientenfeld wird vor der eigentlichen Messung zu einer Ortskodierung über die sich einstellende Phasenlage genutzt. In einer dazu zweckmäßigen Ausgestaltung wird nach Abschalten des magnetischen Gradientenfeldes ein zweites gleiches magnetisches Gradientenfeld umgekehrten Vorzeichens mit derselben Zeitspanne erzeugt. Dieses Verfahren ermöglicht die Durchführung einer Vermessung von Flussprofilen oder Gefäßstrukturen.

Dabei wird für eine vorbestimmte Zeitdauer zunächst ein magnetischer Gradient in einer vorgegebenen Raumrichtung, in der der Fluss gemessen werden soll, angelegt. Anschließend wird für die gleiche Zeitdauer derselbe Gradient mit derselben Gradientenstärke, jedoch mit umgekehrtem Vorzeichen angelegt. Anschließend wird die Frequenzverteilung der Quermagnetisierung erfasst. Die ersten beiden Schritte haben den Effekt, dass unbewegte Kleinstpartikel keine Phasenänderung erfahren, da der zweite Schritt für ruhende Partikel den ersten Schritt quasi vollständig rückgängig macht. Sich in Richtung des Gradienten bewegende Partikel erfahren jedoch eine Phasenänderung, die proportional zur zurückgelegten Wegstrecke ist. Die Phasenänderung ist damit ein Maß für die Flussgeschwindigkeit. Insbesondere kann die mittlere Durchflussrate abgeleitet werden. Über diese Maßnahme kann beispielsweise die Durchflussmenge des untersuchten Mediums kontrolliert werden.

Um eine Flussmessung auch mit Partikeln mit großer Streuung der Rotationseigenschaften durchführen zu können, ist eine Kombination mit einem 180°-Spinflip vorteilhaft. Nach dem Refokussierungspuls ergibt sich durch die Drehrichtungsumkehr des rotierenden Magnetfeldes ein Echosignal, dass bei ruhendem Partikel bei genau der doppelten Zeit zwischen Refokussierungspuls und Drehrichtungsumkehr auftritt. Die inzwischen aufgrund der Partikeleigenschaften außer Phase geratenen Kleinstpartikel rotieren nach Drehrichtungsumkehr wieder in Phase, woraus ein in der Intensität wieder angewachsenes Echosignal der superpositionierten Quermagnetisierung resultiert. Wenn sich die Kleinstpartikel oder das Partikelensemble in Richtung des Gradienten bewegt, dann verschiebt sich dieses Echosignal proportional zur Geschwindigkeit, wobei jeglicher Einfluss aufgrund von Partikelstreuung neutralisiert ist. Die gestellte Aufgabe wird erfindungsgemäß durch eine Vorrichtung gemäß des Anspruchs 12 gelöst. Die Messeinrichtung umfasst erfindungsgemäß eine Anzahl von mit ihrer Symmetrieachse senkrecht zur Längsachse ausgerichteter Induktionsspulen. Die Induktionsspulen können insbesondere als Solenoid-Spulen mit mehreren parallelen Windungen ausgestaltet sein. Über die Induktionsspulen wird unmittelbar der zeitliche Verlauf der superpositionierten Quermagnetisierung als Messsignal erfasst. Die Induktionsspannung gibt hierbei die zeitliche Änderung der Quermagnetisierung an.

Weiter bevorzugt ist die Steuervorrichtung eingerichtet, den ersten Magnetfeldgenerator pulsförmig ein- und auszuschalten, und den zweiten Magnetfeldgenerator erst nach einer Verzögerungszeit nach dem Ausschalten des ersten Magnetfeldgenerators einzuschalten. Zum einen wird über die Verzögerungszeit ein Übersprechen des Erregersignals in das Messsignal verhindert. Zum anderen werden über diese Verzögerungszeit freie ungebundene Kleinstpartikel für das Messsignal aufgrund der kürzeren Relaxationszeit der superpositionierten Quermagnetisierung abgetrennt.

Ist die Steuervorrichtung eingerichtet, die Messvorrichtung erst nach einer Totzeit nach dem Abschalten des Fokussierungsfeldes einzuschalten, so wird zusätzlich verhindert, dass durch Induktion das Erregersignal auf das Messsignal überspricht. Dazu kann bevorzugt ein Schaltelement vorgesehen sein, welches während der Totzeit die Messeinrichtung abtrennt oder kurzschließt. Wird der Empfänger vor einem Tiefpass nicht abgetrennt, lässt ein Rechteckimpuls den Tiefpass nachschwingen, wodurch sich eine lange Verzögerungszeit ergibt.

In einer bevorzugten Alternative sind der erste und der zweite Magnetfeldgenerator zu einem Generator zusammengefasst. Dabei umfasst dieser Generator weiter vorteilhaft zwei Paare von senkrecht zueinander angeordneten Spulen, insbesondere Helmholtzspulen. Durch getrennte Ansteuerung eines Paares der Spulen wird es möglich, das gerichtete Fokussierungsfeld zu erzeugen. Alternativ ist es auch möglich, das Fokussierungsfeld durch Einschalten oder durch eine Erhöhung der Feldstärke des rotierenden Magnetfelds zu erzeugen. Hierbei werden die Spulenpaare eingeschaltet bzw. entsprechend stärker bestromt.

Zur Abtrennung der Rotationsfrequenz des rotierenden Magnetfeldes von der Frequenz der Quermagnetisierung ist weiter bevorzugt ein Tiefpassfilter oder ein Bandpassfilter vorgesehen.

Zur Unterdrückung von durch das äußere Magnetfeld erzeugten Induktionsspannungen oder -strömen im Messsignal ist das Eingangssignal des zweiten Magnetfeldgenerators bevorzugt mit dem Ausgangssingal der Messeinrichtung, insbesondere mit dem Spulenausgangssignal, gegenphasig gekoppelt. Diese Kopplung kann beispielsweise induktiv mittels eines Transformators oder dergleichen erfolgen.

Bevorzugt wird auch eine aktive Gegenkopplung zur Verbesserung des Messverfahrens eingesetzt. Dabei werden die Störanteile in der Empfangskette in einer Kalibriermessung erfasst. Diese Störanteile werden dann in der eigentlichen Messung mit entgegengesetztem Vorzeichen direkt in die Empfangskette eingekoppelt. Dazu ist vorteilhafterweise ein Transformator oder allgemein ein HF-Übertrager vorgesehen. Das Einkopplungssignal wird insbesondere mittels eine Digital-Analog-Konverter generiert.

Ausführungsbeispiele der Erfindung werden anhand einer Zeichnung näher erläutert. Dabei zeigen:

Fig.1    Schematisch den Aufbau einer Vorrichtung zur Detektion eines Analyten mittels eines Ensembles von magnetischen Kleinstpartikeln,

Fig. 2   das Abklingverhalten der superpositionierten Quermagnetisierung für zwei Beispielfälle,

Fig. 3   den zeitlichen Verlauf der superpositionierten Quermagnetisierung der Beispielfälle entsprechend Fig. 2 mit Echo-Erzeugung,

Fig. 4   den Feldverlauf der präparierenden und messenden Magnetfelder gemeinsam mit einem Messsignal der superpositionierten Quermagnetisierung und

Fig. 5   den Verlauf der superpositionierten Quermagnetisierung für freie und für gebundene Kleinstpartikel.

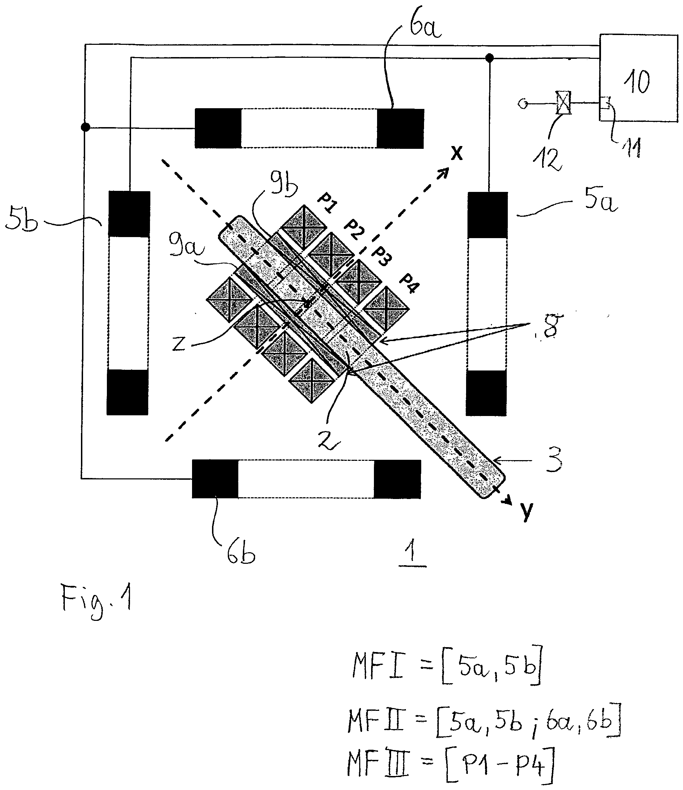

In Fig. 1 ist schematisch eine Vorrichtung 1 zur Bestimmung der Anwesenheit eines Analyten mittels eines Ensembles magnetischer Kleinstpartikel dargestellt. In einem Messvolumen 2 wird hierbei die superpositionierte Quermagnetisierung eines Ensembles von magnetischen Kleinstpartikeln in einer Probe 3 beobachtet. Die Probe 3 ist dabei beispielsweise eine Biopsienadel mit einer Gewebeprobe. Die zugesetzten magnetischen Kleinstpartikel weisen eine funktionalisierte Oberfläche auf, die an Rezeptoren pathologischer Zellen ankoppelt.

Zum Erzeugen eines um die Längsachse z rotierenden äußeren Magnetfeldes sind ein erstes Helmholtzspulenpaar 5a, 5b und ein zweites Helmholtzspulenpaar 6a, 6b vorgesehen. Die beiden Helmholtzspulenpaare 5a,b und 6a,b sind hierbei orthogonal zueinander angeordnet. Das um die Längsachse z rotierende Magnetfeld ergibt sich durch wechselseitige Bestromung der beiden Helmholtzspulenpaare 5a,b und 6a,b.

Als eine Messeinrichtung 8 zur Erfassung des zeitlichen Verlaufs der superpositionierten Quermagnetisierung sind zwei Induktionsspulen 9a und 9b seitlich neben dem Messvolumen 2 angeordnet. Die Symmetrieachse der beiden Induktionsspulen 9a,b steht senkrecht zur Längsachse z.

Zur Steuerung der Helmholtzspulenpaare 5a,b und 6a,b und zum Auslesen der Messeinrichtung 8 ist eine Steuervorrichtung 10 vorgesehen.

Entlang der Probenrichtung y sind des Weiteren eine Anzahl von Spulenpaaren P1 bis P4 angeordnet. Diese Spulenpaare P1 bis P4 können zum Erzeugen eines Fokussierungsfeldes herangezogen werden. Insbesondere kann über eine separate Ansteuerung der vier Spulenpaare P1 bis P4 ein Gradientenfeld und insbesondere ein in y-Richtung wanderndes Gradientenfeld erzeugt werden. Ein solches Gradientenfeld und insbesondere ein solches wanderndes Gradientenfeld dient zu einer spezifischen Präparation der Probe, nämlich insbesondere dem Aufprägen eines ortsabhängigen Phasenverlaufs der rotierenden Magnetisierungen der Kleinstpartikel, wie es für ein Bildgebungsverfahren benötigt wird. Für eine reine Detektion der Anwesenheit eines Analyten kann auf die Spulenpaare P1 bis P4 verzichtet werden.

Zur Detektion eines Analyten, beispielsweise einer pathologischen Zelle oder dergleichen, wird über die Steuervorrichtung 10 zunächst das Hemholtzspulenpaar 5a,5b pulsförmig bestromt, so dass am Messvolumen 2 für eine kurze Zeitspanne ein gerichtetes magnetisches Fokussierungsfeld herrscht. Das Fokussierungsfeld zeigt hierbei in eine Richtung parallel zur Hauptsymmetrieachse des Helmholtzspulenpaares 5a, 5b. Die Bestromung des Helmholtzspulenpaares 5a, 5b ist hierbei derart gewählt, dass die Feldstärke des magnetischen Fokussierungsfeldes die Magnetisierungen der magnetischen Kleinstpartikel zu einer makroskopischen Magnetisierung ausrichtet. Um unerwünschte Frequenzanteile im Messsignal zu unterdrücken, wird das Helmholtzspulenpaar 5a, 5b derart bestromt, dass ein Sinc-Puls förmiges Magnetfeld als Fokussierungsfeld erzeugt ist.

Nach einer Verzögerungszeit bestromt die Steuervorrichtung 10 beide Helmholtzspulenpaare 5a, b und 6a, b abwechselnd, so dass ein um die Längsachse z rotierendes äußeres Magnetfeld im Messvolumen 2 generiert wird. Die Feldstärke und die Rotationsfrequenz dieses Magnetfelds sind derart gewählt, dass die Magnetisierungen der magnetischen Kleinstpartikel asynchron rotieren. Die Verzögerungszeit zwischen dem Anlegen des Fokussierungsfeldes und dem Anlegen des rotierenden Magnetfeldes ist derart eingestellt, dass die makroskopische Magnetisierung der freien Kleinstpartikel durch Diffusionseffekte etc. bei Einschalten des rotierenden Magnetfeldes bereits zerfallen ist. Da der Zerfall der makroskopischen Magnetisierung der gebundenen Kleinstpartikel langsamer ist, ist zumindest ein Restbetrag der Magnetisierung der gebundenen Kleinstpartikel zum Zeitpunkt des Einschaltens des rotierenden Magnetfelds noch erhalten.

Nach Einschalten des rotierenden Magnetfeldes beginnen die Magnetisierungen der gebundenen Kleinstpartikel asynchron zur rotieren, so dass mittels der beiden Induktionsspulen 9a, b der zeitliche Verlauf der superpositionierten Quermagnetisierung in der x, y - Ebene beobachtet werden kann. Ist der spezifische Analyt in der Probe nicht vorhanden, so wird durch Anlegen des auslesenden rotierenden Magnetfeldes kein Signal generiert. Ist der Analyt vorhanden, so führt dies zur Bildung gebundener magnetischer Kleinstpartikel. Mit Einschalten des Auslesefeldes ist als Messwert eine superpositionierte Quermagnetisierung erfassbar. Diese wird über die Induktionsspulen 9a, b als ein zeitlich variierendes Messsignal erfasst, da die superpositionierte Quermagnetisierung mit einer mittleren asynchronen Rotationsfrequenz in der x, y - Ebene rotiert. Das Vorhandensein eines Messsignals zeigt insofern das Vorhandensein des Analyten unmittelbar an. Insbesondere ist eine rasche und aussagekräftige Methode gegeben, um beispielsweise feststellen zu können, ob ein entnommenes Gewebe pathologisch ist.

Das beschriebene Messverfahren mit der in Fig. 1 dargestellten Vorrichtung 1 ist dadurch gekennzeichnet, dass als ein erster Magnetfeldgenerator MFI das Helmholtzspulenpaar 5a, b verwendet wird. Als auslesender Magnetfeldgenerator MFII werden die Helmholtzspulenpaare 5a, b und 6a, b eingesetzt. Durch eine entsprechende Bestromung resultiert das gewünschte rotierende äußere Magnetfeld. Alternativ zum Helmholtzspulenpaar 5a, b kann als ein erster Magnetfeldgenerator MFIII die Abfolge der Spulenpaare P1 bis P4 verwendet werden. Die Anzahl der Spulenpaare ist hier beliebig.

Zum Kurzschließen oder Trennen der Messspulen 9a, b ist ein entsprechendes, von der Steuervorrichtung 10 betätigbares Schaltelement 11 vorgesehen. Die Abtrennung des Erregersignals vom Messsignal übernimmt ein Tiefpassfilter 12.

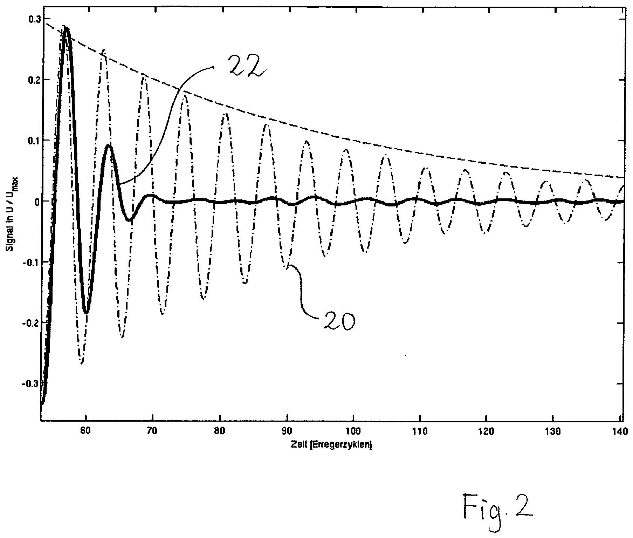

In Fig. 2 ist zur Erläuterung der zeitliche Verlauf einer superpositionierten Quermagnetisierung 20 eines simulierten ersten Ensembles von magnetischen Kleinstpartikeln dargestellt. Bei dieser Simulation werden Diffusionseffekte berücksichtigt, die zu einem Kohärenzverlust der einzelnen asynchron rotierenden Magnetisierungen der Kleinstpartikel führen. Nach Präparation der Probe durch ein magnetisches Fokussierungsfeld geeigneter Stärke beginnen bei Anlegen eines äußeren rotierenden Magnetfeldes entsprechender Feldstärke und Rotationsfrequenz die Magnetisierungen der Kleinstpartikel asynchron zu rotieren. Die über die superpositionierte Quermagnetisierung beobachtbare mittlere asynchrone Rotationsfrequenz ist in Fig. 2 unmittelbar aus dem Verlauf der Quermagnetisierung ersichtlich. Durch Kohärenzverlust aufgrund der Diffusion wird mit jeder Schwingung die Amplitude der beobachtbaren superpositionierten Quermagnetisierung geringer. Die gestrichelt eingezeichnete Einhüllende zeigt die durch Diffusionseffekte bedingte Relaxationszeit.

Daneben ist der Verlauf der superpositionierten Quermagnetisierung 22 eines simulierten Ensembles aus magnetischen Kleinstpartikeln eingezeichnet, wobei die Partikel in ihren Eigenschaften eine 20%-ige Variation aufweisen. Zusätzlich ist auch dem rotierenden äußeren Magnetfeld eine 20%-ige Variation der Feldstärke innerhalb des Messvolumens zugewiesen.

Es wird deutlich erkennbar, dass die Relaxationszeit des zweiten Partikelensembles gegenüber dem ersten Partikelensemble deutlich verkürzt ist. Neben Diffusionseffekten führen die Variation der Partikeleigenschaften und die Feldinhomogenitäten zu einer sehr viel rascheren Dephasierung der einzelnen Magnetisierungen der Kleinstpartikel und somit zu einem rascheren Verfall der superpositionierten Quermagnetisierung.

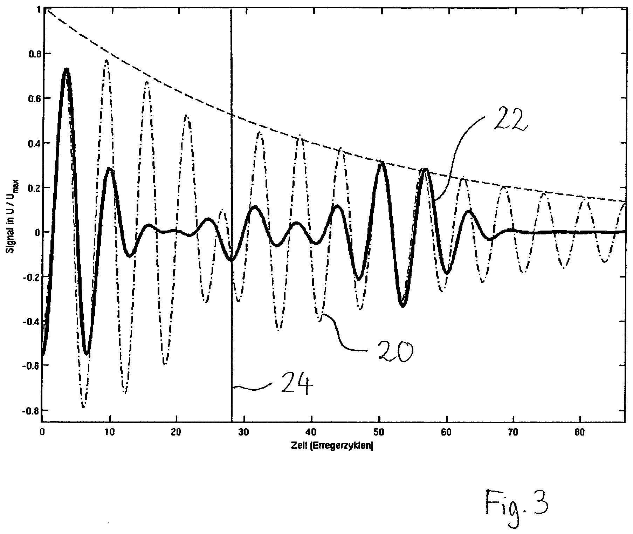

In Fig. 3 ist der Verlauf der superpositionierten Quermagnetisierungen 20 bzw. 22 entsprechend Fig. 2 dargestellt, wobei jedoch zum Zeitpunkt 24 die Drehrichtung des äußeren rotierenden Magnetfeldes umgekehrt wird. Fig. 3 zeigt demnach die Durchführung eines Echo-Verfahrens. Durch die Umkehr der Drehrichtung des äußeren rotierenden Magnetfeldes kann die Dephasierung der Magnetisierung der Kleinstpartikel teilweise wieder rückgängig gemacht werden. Zu einem gewissen Zeitpunkt nach Drehrichtungsumkehr 24 rotiert ein gewisser Anteil der Magnetisierung der Kleinstpartikel wieder in Phase. Das beobachtbare Messsignal der superpositionierten Quermagnetisierung wächst erneut an.

Insbesondere die rascher zerfallende Quermagnetisierung des zweiten Partikelensembles kann durch Anwendung des Echo-Verfahrens sehr viel genauer und mit geringeren Messfehlern ausgewertet werden. Das Echo-Verfahren liefert zeitlich ein breiteres Messsignal, was im Frequenzraum zu einer schmäleren Bandbreite führt.

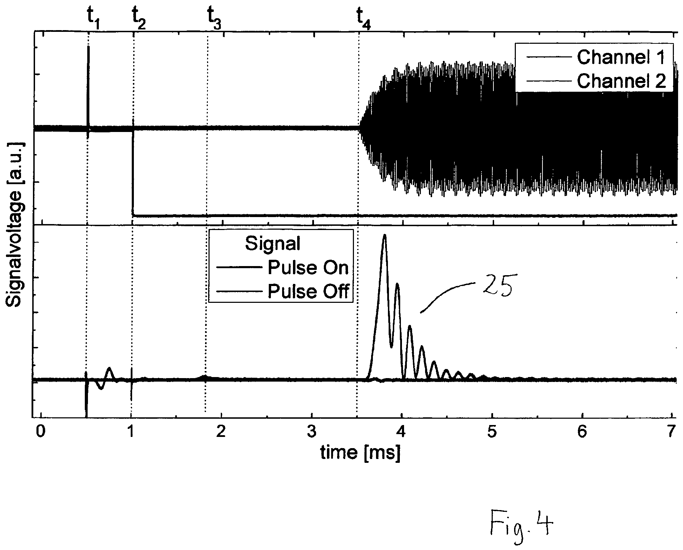

In Fig. 4 ist in der oberen Bildhälfte der Verlauf der magnetischen Felder zur Präparation und zum Auslesen einer Probe dargestellt. In der unteren Bildhälfte ist der zeitliche Verlauf der beobachteten superpositionierten Quermagnetisierung 25 dargestellt. Durchgeführt wird das Echo-Verfahren.

Zum Verständnis von Fig. 4 kann Bezug genommen werden auf die Vorrichtung 1 gemäß Fig. 1, die zur Durchführung des gezeigten Messverfahrens geeignet ist. Als Probe werden magnetische Eisenpartikel einer Größe von etwa 100nm in Wasser untersucht, die dazu neigen, Eisen-Cluster mit einer Größe von etwa 100 $\mu$m zu bilden.

Zum Zeitpunkt $t_1$ wird das Helmholtzspulenpaar 5a, b entsprechend Fig. 1 pulsförmig bestromt. Es resultiert ein gerichtetes gepulstes Magnetfeld, mit dem die Magnetisierungen der Eisenpartikel ausgerichtet werden. Das pulsförmig erzeugte gerichtete Magnetfeld weist in etwa eine Halbwertsbreite von 20$\mu$s auf. In der Darstellung ist eine Struktur des Magnetfeldpulses nicht erkennbar.

Bis zum Zeitpunkt $t_2$ bleiben die Induktionsspulen 9a, b kurzgeschlossen. Am Zeitpunkt $t_2$ werden die Induktionsspulen 9a, b aktiviert. Durch diese Maßnahme wird es sichergestellt, dass kein Erregersignal induktiv in das Messsignal übertreten kann. Die Abschaltdauer des Kurzschlusses der Induktionsspulen bedingt eine Verzögerung deren voller Empfangsbereitschaft bis zum Zeitpunkt $t_3$.

Zum Zeitpunkt $t_4$, also nach einer Verzögerungszeit nach dem Ausschalten des Fokussierungsfeldes werden die Helmholtzspulenpaare 5a, b und 6a, b jeweils mit unterschiedlichen Frequenzen bestromt. Beispielsweise wird auf eines der beiden Spulenpaare 5a, b eine Frequenz von 50kHz und auf das andere Spulenpaar 6a, b eine Frequenz von 58kHz geschaltet. Durch diese spezifische Bestromung resultiert ein rotierendes äußeres Magnetfeld im Messvolumen, welches periodisch seine Drehrichtung umkehrt. Die Frequenz der Drehrichtungsumkehr ist hierbei durch die Schwebungsfrequenz, also durch (50kHz - 58kHz)/2 = 4kHz gegeben. Diese Schwebungsfrequenz ist als eine Echofrequenz aufzufassen, mit der die dephasierte Magnetisierungen der Kleinstpartikel zumindest teilweise wieder in Phase geraten.

Durch das Fokussierungsfeld resultiert eine makroskopische Magnetisierung durch Ausrichten der magnetischen Momente der einzelnen Eisenpartikel. Die Magnetisierung der freien Eisenpartikel relaxiert innerhalb der eingestellten Verzögerungszeit. Die makroskopische Magnetisierung der zu Eisenclustern zusammengeballten Eisenpartikel bleibt aufgrund der verlangsamten Relaxation aufgrund der zunehmenden Größe bis zum Einschalten des rotierenden Magnetfeldes erhalten. Nach Einschalten des auslesenden rotierenden äußeren Magnetfelds beginnen die Magnetisierungen der einzelnen Eisencluster asynchron zu rotieren. Dazu ist die Feldstärke des rotierenden Magnetfeldes entsprechend eingestellt.

Aufgrund der mechanischen und magnetischen Kopplung der einzelnen Eisenpartikel im Eisen-Cluster zerfällt die superpositionierte Quermagnetisierung nach Anlegen des äußeren rotierenden Magnetfeldes rasch. Die magnetischen Momente der einzelnen Eisenpartikel orientieren sich zunehmend stochastisch zueinander. Die anfänglich noch in Phase rotierenden Magnetisierungen der Eisen-Cluster geraten außer Phase. Auch die Echo-Signale des Verlaufs der superpositionierten Quermagnetisierung 25 klingen rasch ab. Die einzelnen Spitzen/Maxima in dem Verlauf der Quermagnetisierung 25 spiegeln die Echofrequenz wieder. Die mittlere asynchrone Rotationsfrequenz ist in dieser Darstellung nicht aufgelöst.

Es wird deutlich erkennbar, dass mit dem geschilderten Verfahren eine Verclusterung der Eisen-Partikel beeindruckend rasch und sicher feststellbar ist. Ein vergleichbares Signal ergibt sich, wenn die Oberflächen der magnetischen Kleinstpartikel entsprechend präpariert sind, so dass eine Ankopplung an einen spezifischen Partner, nämlich den zu untersuchenden Analyten, oder eine Verclusterung über diesen stattfindet.

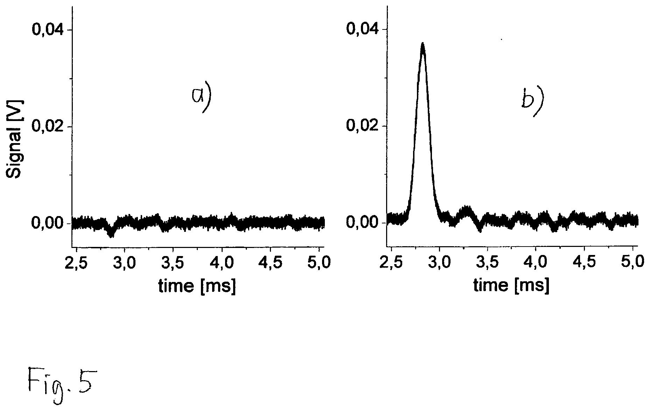

In den Fig. 5 ist der Verlauf der superpositionierten Quermagnetisierung, wie sie entsprechend Fig. 4 gemessen wurde, für zwei verschiedene Probenpräparationen dargestellt. Als Probe werden Magnetit-Kleinstpartikel mit einem Durchmesser von 100 nm in Wasser betrachtet. Die Magnetit-Kleinstpartikel weisen eine anionische Umhüllung auf und eignen sich insbesondere zur Adsorption von Biomolekülen. Derartige Kleinstpartikel sind beispielsweise unter dem Handelsnamen fluidMAG bei der Chemiezell GmbH erhältlich. Es handelt sich hierbei um superparamagnetische Nanopartikel.

In Wasser führt die anionische Umhüllung der Kleinstpartikel dazu, dass eine Verclusterung nicht stattfindet. Wird insofern die superpositionierte Quermagnetisierung wie vorbeschrieben zu Fig. 4 gemessen, so ist bei Einschalten des auslesenden rotierenden äußeren Magnetfelds bei etwa 2,5 ms die makroskopische Magnetisierung dieser Kleinstpartikel bereits relaxiert. Entsprechend ist gemäß Fig. 5a kein Signal der superpositionierten Quermagnetisierung zu beobachten.

Werden durch Zugabe einer Pufferlösung Kationen in das Wasser eingebracht, so koppeln die anionischen Kleinstpartikel über diese Kationen aus der Lösung miteinander, und bilden einen Partikelcluster. Die Relaxation der makroskopischen Magnetisierung dieser Cluster wird aufgrund der Größenzunahme oder aufgrund eines geänderten Formfaktors gehemmt. Bei Anlegen des auslesenden rotierenden äußeren Magnetfelds resultiert eine messbare superpositionierte Quermagnetisierung. Dies ist deutlich in Fig. 5b ersichtlich. Aufgrund der Darstellung sowie aufgrund von Messartefakten ist eine Echo-Fluktuation des Messsignals nicht erkennbar.

Die Figuren 5a und 5b zeigen eindrucksvoll, dass das vorbeschriebene Verfahren in der Lage ist, das Vorhandensein eines Analyten rasch und mit großer Genauigkeit zu detektieren. Durch entsprechende Präparation können die Kleinstpartikel als Sonden zur Detektion des spezifischen Analyten eingesetzt werden.

Bezugszeichenliste

| | |
|---|---|
| 1 | Vorrichtung |
| 2 | Messvolumen |
| 3 | Probe |
| 5a,b | erstes Spulenpaar |
| 6a,b | zweites Spulenpaar |
| 8 | Messeinrichtung |
| 9a,b | Induktionsspulen |
| 10 | Steuervorrichtung |
| 11 | Schaltelement |
| 12 | Tiefpassfilter |
| 20 | Quermagnetisierung (Diffusion) |
| 22 | Quermagnetisierung (Inhomogenitäten, Partikelstreuung) |
| 24 | Feldumkehr |
| 25 | Echosignal |

| | |
|---|---|
| MFI, MFIII | erster Magnetfeldgenerator |
| MFII | zweiter Magnetfeldgenerator |
| P1-P4 | Spulen |
| t1 - t4 | Zeiten |
| x | Querachse |
| y | Querachse |
| z | Längsachse |

**Patentansprüche**

1. Verfahren zur Bestimmung der Anwesenheit eines Analyten mittels einer Verteilung magnetischer Kleinstpartikel, an welche der Analyt koppelt, wobei

   - mittels eines äußeren magnetischen Fokussierungsfeldes die Magnetisierungen der Kleinstpartikel zueinander ausgerichtet werden,
   - nach Abschalten des Fokussierungsfeldes mittels eines um eine Längsachse (z) rotierenden äußeren Magnetfeldes geeigneter Feldstärke und Rotationsfrequenz die Magnetisierungen der Kleinstpartikel in eine zum Magnetfeld asynchrone Rotation versetzt werden,

**dadurch gekennzeichnet, dass**

   - der zeitliche Verlauf der auf eine Ebene senkrecht zur Rotationsachse projizierten superpositionierten Quermagnetisierung des Partikelensembles mittels einer Anzahl von mit ihrer Symmetrieachse senkrecht zur Längsachse ausgerichteter Induktionsspulen erfasst wird, und
   - aus dem erfassten zeitlichen Verlauf mittels einer Analyse der Frequenzanteile der Quermagnetisierung auf die Anwesenheit des Analyten geschlossen wird.

2. Verfahren nach Anspruch 1,
   wobei der zeitliche Verlauf der Quermagnetisierung erst mit einer Verzögerungszeit nach dem Abschalten des Fokussierungsfeldes erfasst wird.

3. Verfahren nach Anspruch 1 oder 2,
   wobei aus dem Verlauf der Quermagnetisierung die zeitliche Ableitung gebildet wird, und aus der Ableitung auf die Anwesenheit des Analyten geschlossen wird.

4. Verfahren nach einem der vorhergehenden Ansprüche,
   wobei die Rotationsrichtung des rotierenden Magnetfeldes mit einer Echofrequenz in der Richtung umgekehrt wird.

5. Verfahren nach einem der vorhergehenden Ansprüche,
   wobei das rotierende Magnetfeld mittels zweier senkrecht zueinander stehender Spulenpaare (5a, 5b, 6a, 6b) oder Spulenanordnungen erzeugt wird, die mit zeitlich variierenden Phasendifferenzen angesteuert werden.

6. Verfahren nach einem der vorhergehenden Ansprüche,
   wobei das Fokussierungsfeld durch das rotierende Magnetfeld erzeugt wird.

7. Verfahren nach einem der vorhergehenden Ansprüche,
   wobei aus dem erfassten zeitlichen Verlauf der Quermagnetisierung auf die Konzentration des Analyten geschlossen wird.

**8.** Verfahren nach einem der vorhergehenden Ansprüche,
wobei den magnetischen Kleinstpartikeln funktionale Gruppen angelagert werden, die sich an den Analyten binden.

**9.** Verfahren nach einem der vorhergehenden Ansprüche,
wobei die physikalischen oder chemischen Umgebungsparameter des Analyten nachgewiesen werden.

**10.** Verfahren nach einem der vorhergehenden Ansprüche,
wobei zu einer Phasenkodierung ein magnetisches Gradientenfeld für eine vorgegebene Zeitspanne erzeugt wird.

**11.** Verfahren nach Anspruch 10,
wobei nach Abschalten des magnetischen Gradientenfeldes ein zweites gleiches magnetisches Gradientenfeld umgekehrten Vorzeichens mit derselben Zeitspanne erzeugt wird, und wobei danach die Quermagnetisierung ermittelt und ausgegeben wird.

**12.** Vorrichtung (1) zur Bestimmung der Anwesenheit eines Analyten mittels magnetischer Kleinstpartikel, an welche der Analyt koppelt, mit einem ersten Magnetfeldgenerator (MFI, MFIII) zur Erzeugung eines magnetischen Fokussierungsfeldes, einem zweiten Magnetfeldgenerator (MFII) zur Erzeugung eines um eine Längsachse (z) rotierenden Magnetfeldes, mit einer Messeinrichtung (8) zur Erfassung des zeitlichen Verlaufs der auf eine Ebene senkrecht zur Rotationsachse projizierten superpositionierten Quermagnetisierung, wobei die Messeinrichtung (8) eine Anzahl von mit ihrer Symmetrieachse senkrecht zur Längsachse ausgerichteter Induktionsspulen (9a, 9b) umfasst, und mit einer Steuervorrichtung (10), die eingerichtet ist, die Magnetfeldgeneratoren (MFI, MFII, MFIII) entsprechend einem Verfahren nach einem der vorhergehenden Ansprüche anzusteuern und den zeitlichen Verlauf der Quermagnetisierung oder eine hieraus abgeleitete Größe zur Bestimmung der Anwesenheit des Analyten auszugeben.

**13.** Vorrichtung (1) nach Anspruch 12,
wobei die Steuervorrichtung (10) eingerichtet ist, den ersten Magnetfeldgenerator (MFI, MFIII) pulsförmig ein- und auszuschalten, und den zweiten Magnetfeldgenerator (MFII) erst nach einer Verzögerungszeit nach dem Ausschalten des ersten Magnetfeldgenerators (MFI, MFIII) einzuschalten.

**14.** Vorrichtung (1) nach Anspruch 12 oder 13,
wobei der erste und der zweite Magnetfeldgenerator (MFI; MFII; MFIII) zu einem Generator zusammengefasst sind, und dieser Generator zwei Paare von senkrecht zueinander angeordneten Helmholtzspulen (5a, 5b, 6a, 6b) umfasst.

**15.** Vorrichtung (1) nach einem der Ansprüche 12 bis 14, wobei das Eingangssignal des zweiten Magnetfeldgenerators (MFII) mit dem Ausgangssignal der Messeinrichtung (8) gegenphasig gekoppelt ist.

**Claims**

**1.** Method for determining the presence of an analyte by means of a distribution of small magnetic particles to which the analyte couples, wherein

- the magnetisations of the small particles are aligned with one another by means of an outer magnetic focusing field,
- after the focusing field has been switched off, the magnetisations of the small particles are set in rotation asynchronously to the magnetic field by means of an outer magnetic field rotating around a longitudinal axis (z) of suitable field strength and rotation frequency,

**characterised in that**,

- the temporal progression of the superpositioned transverse magnetisation of the particle set which is projected onto a plane perpendicular to the axis of rotation is detected by means of a number of induction coils which are aligned perpendicular to the longitudinal axis with their axis of symmetry, and
- the presence of the analyte is deduced from the detected temporal progression by means of an analysis of the frequency components of the transverse magnetisation.

**2.** Method according to claim 1,
wherein the temporal progression of the transverse magnetisation is only detected with a time delay after the focusing field has been switched off.

**3.** Method according to claim 1 or 2,
wherein the time derivative is formed from the temporal progression of the transverse magnetisation, and the presence of the analyte is deduced from the derivative.

**4.** Method according to one of the preceding claims,
wherein the direction of rotation of the rotating magnetic field is reversed in direction with an echo frequency.

**5.** Method according to one of the preceding clams,
wherein the rotating magnetic field is generated by means of two mutually perpendicular coil pairs or coil arrangements (5a, 5b, 6a, 6b), which are controlled with time-varying phase differences.

6. Method according to one of the preceding claims, wherein the focusing field is generated by the rotating magnetic field.

7. Method according to one of the preceding claims, wherein the concentration of the analyte is deduced from the detected temporal progression of the transverse magnetisation.

8. Method according to one of the preceding claims, wherein functional groups which bind to the analytes are added to the small magnetic particles.

9. Method according to one of the preceding claims, wherein the physical or chemical environmental parameters of the analyte are established.

10. Method according to one of the preceding claims, wherein a magnetic gradient field is generated for a predetermined time interval for phase encoding.

11. Method according to claim 10, wherein a second identical magnetic gradient field of the opposite sign with the same time interval is produced after the magnetic gradient field is switched off, and wherein after this the transverse magnetisation is identified and output.

12. Device (1) for determining the presence of an analyte by means of small magnetic particles to which the analyte couples, having a first magnetic field generator (MFI, MFIII) for generating a magnetic focusing field, a second magnetic field generator (MFII) for generating a magnetic field which rotates around a longitudinal axis (z), having a measuring device (8) for detecting the temporal progression of the superpositioned transverse magnetisation which is on a plane perpendicular to the axis of rotation, wherein the measuring device (8) comprises a number of induction coils (9a, 9b) which are aligned perpendicular to the longitudinal axis with their axis of symmetry, and having a control device (10) which is configured to control the magnetic field generators (MFI, MFII, MFIII) in accordance with a method according to one of the preceding claims and to output the temporal progression of the transverse magnetisation or a value derived therefrom for determining the presence of the analyte.

13. Device (1) according to claim 12, wherein the control device (10) is configured to switch the first magnetic field generator (MFI, MFIII) on and off in pulses, and to switch on the second magnetic field generator (MFII) only after a time delay after the first magnetic field generator (MFI, MFIII) has been switched off.

14. Device (1) according to claim 12 or 13, wherein the first and the second magnetic field generator (MFI; MFII; MFIII) are combined to form a generator, and this generator comprises two pairs of Helmholtz coils (5a, 5b, 6a, 6b) which are arranged perpendicular to one another.

15. Device (1) according to one of claims 12 to 14, wherein the input signal of the second magnetic field generator (MFII) is coupled in antiphase to the output signal of the measuring device (8).

**Revendications**

1. Procédé de détermination de la présence d'un analyte au moyen d'une répartition de petites particules magnétiques auquel l'analyte se couple, dans lequel

   - les magnétisations des petites particules sont orientées les unes vers les autres au moyen d'un champ de focalisation magnétique externe,
   - après arrêt du champ de focalisation, les magnétisations des petites particules sont amenées dans une rotation asynchrone par rapport au champ magnétique au moyen d'un champ magnétique externe, d'une intensité et d'une fréquence de rotation appropriées, tournant autour d'un axe longitudinal (z),

   **caractérisé en ce que**

   - le déroulement temporel de la magnétisation transversale superposée projetée sur un plan perpendiculairement à l'axe de rotation est enregistré au moyen d'une pluralité de bobines d'induction orientées perpendiculairement à l'axe longitudinal avec leur axe de symétrie, et
   - à partir du déroulement temporel enregistré, la présence de l'analyte est déduite au moyen d'une analyse des composantes fréquentielles de la magnétisation transversale.

2. Procédé selon la revendication 1, dans lequel le déroulement temporel de la magnétisation transversale n'est enregistré qu'avec un temps de retard après l'arrêt du champ de focalisation.

3. Procédé selon la revendication 1 ou 2, dans lequel la dérivée temporelle est formée à partir du déroulement de la magnétisation transversale et la présence de l'analyte est déduite de la dérivée.

4. Procédé selon l'une quelconque des revendications précédentes, dans lequel le sens de rotation du champ magnétique rotatif est inversé au moyen d'une fréquence renvoyée dans ce sens.

5. Procédé selon l'une quelconque des revendications précédentes, dans lequel le champ magnétique rotatif est généré au moyen de deux paires de bobines (5a, 5b, 6a, 6b) perpendiculaires l'une à l'autre ou d'ensembles de bobines, qui sont commandés à des différences de phase variant dans le temps.

6. Procédé selon l'une quelconque des revendications précédentes, dans lequel le champ de focalisation est généré par le champ magnétique rotatif.

7. Procédé selon l'une quelconque des revendications précédentes, dans lequel la concentration de l'analyte est déduite du déroulement temporel enregistré de la magnétisation transversale.

8. Procédé selon l'une quelconque des revendications précédentes, dans lequel des groupes fonctionnels se liant à l'analyte sont fixés sur les petites particules magnétiques.

9. Procédé selon l'une quelconque des revendications précédentes, dans lequel les paramètres environnementaux physiques ou chimiques de l'analyte sont établis.

10. Procédé selon l'une quelconque des revendications précédentes, dans lequel un champ de gradient magnétique est généré pendant un laps de temps prédéfini de manière à obtenir un codage de phase.

11. Procédé selon la revendication 10, dans lequel un deuxième champ de gradient magnétique identique de signe inverse est généré pendant le même laps de temps après arrêt du champ de gradient magnétique, et la magnétisation transversale est identifiée et délivrée après cela.

12. Dispositif (1) pour la détermination de la présence d'un analyte au moyen de petites particules magnétiques, auxquelles l'analyte se couple, comprenant un premier générateur de champ magnétique (MFI, MFIII) pour la génération d'un champ de focalisation magnétique, un deuxième générateur de champ magnétique (MFII) pour la génération d'un champ magnétique tournant autour d'un axe longitudinal (z), un dispositif de mesure (8) pour l'enregistrement du déroulement temporel de la magnétisation transversale superposée projetée sur un plan perpendiculairement à l'axe de rotation, dans lequel le dispositif de mesure (8) comporte un certain nombre de bobines d'induction (9a, 9b) orientées perpendiculairement à l'axe longitudinal avec leur axe de symétrie, et un dispositif de commande (10) qui est conçu pour commander les générateurs de champ magnétique (MFI, MFII, MFIII) conformément à un procédé selon l'une quelconque des revendications précédentes, et pour délivrer le déroulement temporel de la magnétisation transversale ou une grandeur déduite de celui-ci pour la détermination de la présence de l'analyte.

13. Dispositif (1) selon la revendication 12, dans lequel le dispositif de commande (10) est conçu pour démarrer et arrêter par impulsions le premier générateur de champ magnétique (MFI, MFIII), et pour démarrer le deuxième générateur de champ magnétique (MFII) qu'après un temps de retard après l'arrêt du premier générateur de champ magnétique (MFI, MFIII).

14. Dispositif (1) selon la revendication 12 ou 13, dans lequel le premier et le deuxième générateur de champ magnétique (MFI ; MFII ; MFIII) sont réunis de manière à former un générateur, et ce générateur comporte deux paires de bobines de Helmholtz (5a, 5b, 6a, 6b) agencées perpendiculairement l'une à l'autre.

15. Dispositif (1) selon l'une quelconque des revendications 12 à 14, dans lequel le signal d'entrée du deuxième générateur de champ magnétique (MFII) est couplé en opposition de phase au signal de sortie du dispositif de mesure (8).

Fig. 1

$$MF\,I = [5a, 5b]$$

$$MF\,II = [5a, 5b ; 6a, 6b]$$

$$MF\,III = [P1 - P4]$$

Fig. 2

Fig. 3

Fig. 4

Fig. 5

**EP 2 553 455 B1**

**IN DER BESCHREIBUNG AUFGEFÜHRTE DOKUMENTE**

**In der Beschreibung aufgeführte Patentdokumente**

- US 20080220411 A1
- WO 2009037636 A